(19)

Europäisches
Patentamt
European
Patent Office
Office européen
des brevets

(11)     EP 3 088 884 A1

(12)          **EUROPEAN PATENT APPLICATION**

(43) Date of publication:
    **02.11.2016  Bulletin 2016/44**

(51) Int Cl.:
    ***G01N 29/04*** (2006.01)          ***G01N 29/11*** (2006.01)
    ***G01N 29/34*** (2006.01)          ***G01N 33/38*** (2006.01)

(21) Application number: **16167381.9**

(22) Date of filing: **28.04.2016**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **MA MD**

(30) Priority: **28.04.2015  EP 15165446**

(71) Applicant: **Uniper Technologies Limited
    Ratcliffe-on-Soar
    Nottingham NG11 OEE (GB)**

(72) Inventor: **Brett, Colin Robert
    Nottingham NG2 6LH (GB)**

(74) Representative: **Glawe, Delfs, Moll
    Partnerschaft mbB
    von Patent- und Rechtsanwälten
    Postfach 13 03 91
    20103 Hamburg (DE)**

(54)     **METHOD AND SYSTEM FOR ULTRASONICALLY DETERMINING THE CONDITION OF A BUILDING STRUCTURE**

(57)     The invention relates to a method and a system for determining the condition of a building structure with at least three layers, the structure comprising a layer of grout between two layers of steel, in an offshore wind turbine foundation.

The method for determining the condition of a building structure (7) with at least three layers, the structure comprising a layer of grout (5) between two layers of steel, in a wind turbine foundations (1), with continuous layers and seamless interfaces between the layers in undamaged condition, comprises the steps of
- exposing a surface area (7') of the structure (7) to ultrasonic energy in a frequency range between 10 and 500 kHz;
- detecting reflections from within the structure (7) at the side of the structure (7) on which the surface area (7') is; and
- analysing the detected reflections to determine faults in the structure;
wherein the ultrasonic energy comprises a spectrum of frequencies.
The Apparatus (10) for conducting the method according to any one of the previous claims, comprises
- an ultrasonic transmitter (15) to expose ultrasonic energy onto a surface area (7') of a structure (7');
- an ultrasonic detector (16) to detect reflections from within the structure (7) at the side of the structure on which the surface area (7') is; and
- an data interface (17) for providing information about the detected reflections for analysis purposes;
wherein the operational range of the transmitter (15) and the detector (16) comprises the frequencies between 10 and 500 kHz.

**Fig. 1**

## Description

The invention relates to a method and a system for determining the condition of a building structure with at least three layers, the structure comprising a layer of grout between two layers of steel, in a wind turbine foundation, particularly in an offshore wind turbine foundation.

For one kind of offshore wind turbine foundations, it is known that a monopile is rammed into the seabed on which a transition piece is mounted that forms the basis for the actual tower of the wind turbine. The transition piece is telescopically arranged on the free end of the monopile that protrudes over the ground of the sea so that the transition piece partially overlaps the monopile. In order to secure the transition piece to the monopile, the gap between the transition piece and the monopile is filled with grout to form a grout connection. The gap usually is 70 mm wide.

Depending on the vertical position of the overlapping section of the transition piece and the monopile, the grout will generally or at least temporarily (i.e. tide-dependently) be below the sea level and thus subjected to water. The general loads and stresses as well as the contact with water generally attacks the grout layer so that the coupling between the transition piece and the monopile is weakened over time. If weakened beyond minimum connection requirements, the grout connection might fail and the transition piece will slip along the monopile telescopically in a downward direction that can lead to serious damage or even destruction of the wind turbine mounted on the foundation.

For protection against serious damage or full destruction of the wind turbine, it is necessary to determine the structural condition of the grout connection to determine the future stability of the wind turbine. However, since the grout is situated between the monopile and the transition piece - both of which are regularly made of steel with a thickness of at least 50 mm each - known conventional non-destructive testing techniques fail when trying to determine the structural condition of the grout connection of offshore wind turbine foundations.

Conventional ultrasonic testing at frequencies of 1 to 10 MHz fails since the ultrasonic waves cannot propagate through the grout without severe attenuation.

US 6,483,777 discloses a method and an apparatus for ultrasonic imaging of a cased well comprising a steel/cement interface, wherein an ultrasonic transmitter is used for emitting a pulsed, collimated acoustic excitation aligned at an angle greater than the shear critical angle to excite a flexural wave in said casing and a receiver displaced axially along the cased well is used to detect the echoes. The echoes are analyzed in the time domain, for example, to determine wave speeds in the cement and analysing such wave speed information to obtain information about cement mechanical properties. However, said shear wave-based excitation combined with time domain analysis does not allow to reliably determine the condition of a building structure in a wind turbine foundation comprising a layer of grout between two layers of steel. For example, ultrasonic shear waves are strongly attenuated in grout, and their propagation at an angel to the surface does not facilitate the measurement.

Radiography would require an extremely active radiation source to penetrate - in total - at least 100 mm of steel and commonly 70 mm of grout. Still, this method would be incapable of e.g. finding cracks at either of the two steel to grout interfaces since such cracks lie in the plane perpendicular to the beam from the radiation source and can thus not be detected.

Any electromagnetic technique can be disregarded since the grout to be tested is very effectively shielded by the steel of the monopile and the transition piece.

It must also be noted that due to the nature of offshore wind turbine foundations the testing needs to take place in situ, i.e. offshore with limited access to the grout section. Therefore, techniques that are based on transmitting a signal through the structure and requiring two apparatuses on each side of the structure to be aligned are generally unsuitable.

It is an object of the present invention to provide a method and an apparatus for determining the condition of a building structure with at least three layers like the grout connection section in an offshore wind turbine foundation that does not suffer the disadvantages of the state of the art, at least not to the same extent.

The object is solved by the features of independent claims 1 and 7. Preferable embodiments are to be found in the dependent claims.

Accordingly, the invention relates to a method for determining the condition of a building structure with at least three layers, the structure comprising a layer of grout between two layers of steel, in a wind turbine foundation, with continuous layers and seamless interfaces between the layers in an undamaged condition, comprising the steps of

- exposing a surface area of the structure to ultrasonic energy in a frequency range between 10 and 500 kHz;
- detecting the reflections from within the structure at the side of the structure on which the surface area is; and
- analysing the detected reflections to determine faults in the structure;

wherein the ultrasonic energy comprises a spectrum of frequencies.

Furthermore, the invention relates to an apparatus for conducting the inventive method, comprising

- an ultrasonic transmitter to expose ultrasonic energy onto a surface area of a structure;

- an ultrasonic detector to detect reflections from within the structure at the side of the structure on which the surface area is; and

- an interface for providing information about the detected reflections for analysis purposes;

wherein the operational range of the transmitter and the detector comprises the frequencies between 10 and 500 kHz.

The invention utilizes the insight that ultrasonic waves with a frequency below 500 kHz are not absorbed by the grout and can therefore propagate through the structure and reflect from the various interfaces within the structure, to be detected by the detector. At the same time, scattering that usually may distort measurements is negligibly small. Both effects in combination allow that the exposing of ultrasonic energy onto the structure and detecting signals therefrom can be achieved on the same side of the structure.

The surface area on which the ultrasonic energy is exposed may be the same area at which the reflections are detected, thereby allowing to investigate the structure beneath said surface area.

It has been found that ultrasound in the mentioned frequency range is generally in a resonance regime for the mentioned purpose of investigating structures with at least three layers (e.g. steel-grout-steel), whereby certain frequencies are preferably transmitted when compared to others and thus allowing the detected reflections to be analysed in order to determine faults in the structure.

The ultrasonic energy comprises a spectrum of frequencies. The spectrum might be a continuous spectrum and/or may cover the whole, or at least one subrange of the given frequency range between 10 and 500 kHz. A preferred frequency range might be between 20 and 200 kHz. In general, for determining a suitable frequency range, it needs to be ensured that every half wavelength $(\frac{\lambda}{2})$ of the frequency within the range fits multiple times within the full thickness of the at least three layers of the structure.

When using a spectrum of frequencies, in the frequency domain there will be a series of resonances that can be detected in the reflections and which help to characterise the structure. Based on the measured reflections, by means of analysis it is possible to draw conclusions regarding the condition of the investigated building structure. In particular, measuring a series of resonances in the reflections and analyzing the measured resonances (i.e the resonant frequencies) allows for interrogating the whole structure simultaneously. Preferably, the analysis comprises determining deviations in the measured reflections from reference reflections. Further, preferably, the analysis comprises identifying faults within the structure based on these deviations. A particular advantage of the inventive method is that the analysis can be carried out using frequency domain information without the need to involve timing information for the detected signals. In other words, the inventive method does not require a time domain analysis, as the analysis of the detected reflections can be carried out entirely in the frequency domain.

The inventive method is especially suitable for determining the condition of a wind turbine foundation, where a transition piece overlaps with a monopile and the gap between those two is filled with grout. Since the transition piece and the monopile are usually made of steel, in this case the structure to be determined comprises a layer of grout between two layers of steel. The layers of steel may each have a nominal thickness between 30 to 70 mm, preferably between 40 to 60 mm, more preferably of 50 mm. The gap between the monopile and the transition piece and thus the nominal thickness of the grout may be between 50 and 90 mm, preferably between 60 and 80 mm, more preferably 70 mm. With these dimensions, the wavelength of the ultrasonic energy used by the invention is in the same order of magnitude as the thickness of the whole structure. In this context it may, however, be noted that while the invention is preferably used with three layer structures, it is not limited to the condition monitoring of three layer structures only but is also suitable for structures with more layers, independent of whether an additional layer is e. g. a paint layer or a structural layer contributing to the strength of the structure.

From analysing the detected reflections, various faults within the structure with three or more layers can be identified. These faults may comprise the absence of a layer material, a crack or de-bonding at the interface between two layers and/or the decrease of structural integrity of one of the layers. Furthermore, using a spectrum of frequencies for the ultrasonic energy allows to probe the whole structure to determine several different faults in the structure simultaneously.

It is preferred that the surface area of the structure, on which the ultrasonic energy is exposed, is submerged in water so that the ultrasonic energy is propagated through the water onto and from the surface area. The water helps to ensure that the ultrasonic energy enters the structure at every point of the surface area evenly while at the same time does not require laborious and wasteful preparatory actions as e.g. using contact gel. In case of offshore wind turbine foundations, the water may be the seawater in which the foundation is sunk.

It is preferred that the ultrasonic energy is propagated onto and/or from the surface area by compression (longitudinal) waves. The attenuation of shear (transverse) waves in grout is extremely high, much higher than the attenuation of longitudinal waves. Therefore, it is preferred that only the longitudinal wave mode is used for propagating the ultrasonic energy onto and from the surface area. Furthermore, if the surface area of the structure, on which the ultrasonic energy is exposed, is submerged in water so that the ultrasonic energy is propagated through the water onto and from the surface area, the longitudinal (compression) wave mode is the only wave mode available, as shear waves cannot propagate through water.

The claimed apparatus is suitable for conducting the inventive method and comprises an ultrasonic transmitter to expose ultrasonic energy onto a surface area of a structure and an ultrasonic detector to detect reflections from within the structure at the side of the structure on which the surface area is. The operational range of both, the transmitter and the detector comprises the frequencies between 10 and 500 kHz, i.e. the frequency range required by the inventive method. The apparatus further comprises an interface for providing information about the detected reflections for analysis purposes. The actual analysis of the detected reflections may thus be done remotely from the claimed apparatus. The ultrasonic transmitter and the ultrasonic detector might be integrated into a single sensor.

In a preferred embodiment the apparatus comprises an analysing module that is connected to data interface of the apparatus. The analysing module is configured for at least partly analysing the detected reflections to determine or help to determine faults in the structure.

Preferably, the transmitter of the apparatus is configured to transmit ultrasonic compression waves onto a surface area of a structure.

Preferably, the apparatus is submersible. It is therefore suitable for inspecting offshore wind turbine foundations, especially the grout-filled overlap between transition piece and monopile. Furthermore, the apparatus being submersible - and thus being intrinsically waterproof - easily allow any space between the transmitter and receiver to be filled with water.

Furthermore it is preferred that the apparatus comprises at least one spacer for ensuring a predetermined distance between transmitter and/or detector and a surface area on which the ultrasonic energy is exposed to. By this, general quality of measurements as well as comparability of measurements can be enhanced. Furthermore, with the help of said at least one spacer, the time for correct position of the apparatus might be shortened. The predetermined distance may be between 0,8 and 1,8 m, preferably between 0,9 and 1,5 m, more preferably 1,0 m, 1,2 m or 1,5 m.

In a preferred embodiment, the apparatus is a diving robot. If the overlap region between a monopile and a transition piece of a wind turbine foundation that is filled with grout is fully submerged at least during high tide, the complete overlap region might be investigated by means of the diving robot investigating one surface area of the structure after the other. Large structural components for positioning and repositioning the sensor to cover the whole overlap region can thus be avoided.

In the following the features, objects and advantages of the present invention are further described by means of exemplary preferred embodiments referring to the accompanying drawings which show:

Figure 1: a schematic view of an offshore wind turbine foundation with a first embodiment of an apparatus according to the invention;

Figure 2: a schematic detail of figure 1 comprising an exemplary wave propagation;

Figure 3: an exemplary diagram of test results originating from an apparatus as shown in figures 1 and 2;

Figures 4a-e: a schematic overview of faults that are detectable by using the invention; and

Figure 5: a second embodiment of an apparatus according to the invention.

In the following, the invention is explained by way of examples of apparatus 10 according to the invention. However, the explanations regarding the functionality of these apparatus 10 also fully illustrate the method according to the invention.

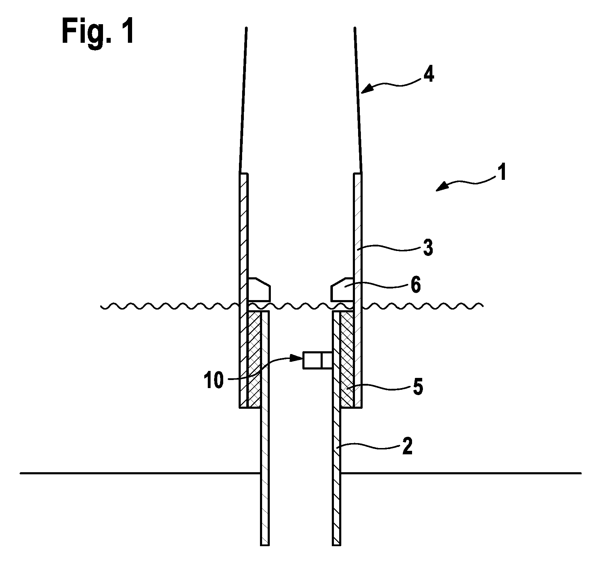

Figure 1 shows a schematic view of an offshore wind turbine foundation 1. The offshore wind turbine foundation 1 comprises a monopile 2 that is rammed into the seabed. On the monopile 2 a transition piece 3 is mounted that forms the basis for the actual tower 4 of the wind turbine. The transition piece 3 is telescopically arranged on the free end of the monopile 2 that protrudes over the ground so that the transition piece 3 partially overlaps the monopile 2. To secure the transition piece 3 to the monopile 2, the gap between the transition piece 3 and the monopile 2 is filled with grout 5 to form a grout connection. In this example, the transition piece 3 and the monopile 2 consist of steel with a nominal thickness of 50 mm. The layer of grout 5 has a nominal thickness of 70 mm.

At least for the tide depicted in figure 1, the overlapping section of the transition piece 3 and the monopile 2 and thus the grout 5 is below the sea level and subjected to seawater at least at the side areas of the overlap region.

For protection against serious damage or full destruction of the wind turbine, special jacking brackets 6 are mounted on the interior surface of the transition piece 3 as safety stoppers to prevent the transition piece from sliding down along the monopile 2 in case the connection between the transition piece 3 and the monopile 2 established by the grout 5 fails.

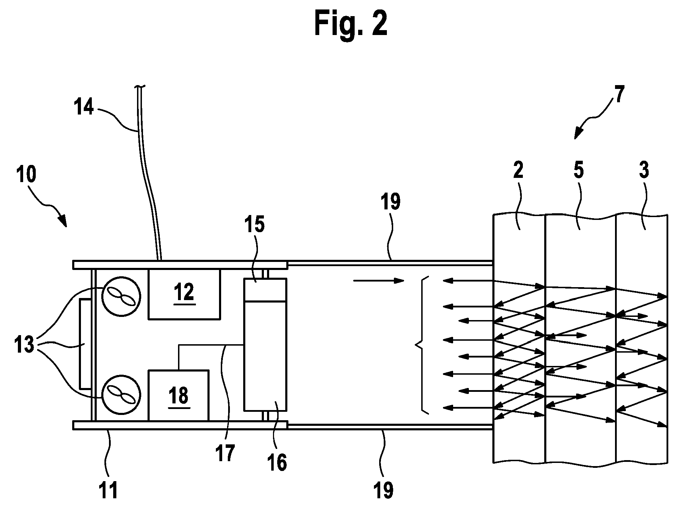

In figure 1 a first embodiment of an apparatus 10 according to the invention is outlined. This apparatus 10 and its functionality, however, will be explained using figure 2 showing a section of figure 1 in more detail.

The overlapping section of the transition piece 3, the monopile 2 and the grout 5 there between forms a three layered structure 7. Even though both, the transition piece 3 as well as the monopile 2 have layers of paint on their outer surfaces, these layers may be neglected in the present explanation. However, it has been found that such layers of paint - whether they are considered to be individual layers or sublayers of other layers - do not affect the general functionality of the apparatus 10 that will be explained below.

The submersible apparatus 10 is designed as a diving robot with a structural frame 11 on which a control device 12 and several propellers 13 are mounted. The supply of electric energy and the communication with a command centre (not shown) is established via cord 14.

On the frame 11, a transmitter 15 and a detector 16 for ultrasonic waves are mounted. The operational range of both, the transmitter 15 and the detector 16 comprises the frequencies between 20 and 200 kHz. The transmitter is arranged 15 to expose ultrasonic energy onto a surface area 7' of the structure 7, while the detector 16 is arranged to detect reflections coming from said surface area 7'. They might also be integrated into a single sensor module.

The detector 16 is connected to a data interface 17 for providing information about the detected reflections for analysis purposes. While the actual analysis of the detected reflection may be done remotely from the apparatus 10, in the given example the apparatus 10 comprises an analysing module 18 that that is connected to data interface 17. The analysing module 18 is configured for at least partly analysing the detected reflections to determine faults in the structure 7. Furthermore, the analysing module 18 is configured to control the transmitter 15, which may be achieved via the data interface 17 already in place or a separate control line (not shown). Results produced by the detector 16 and/or analysing module 18 may be stored locally or transferred to the command centre via the cord 14 in real time.

The apparatus 10 further comprises spacers 19 that help to position the apparatus 10 and thus the transmitter 15 and detector 16 in regard to the surface area 7' of the structure 7. The apparatus 10 may thus be moved towards the structure 7 until the spacers 19 are in full contact with the surface of the structure 7. Afterwards, at least some of the propellers 13 push the apparatus 10 further towards the structure thus establishing a frictional connection between the apparatus 10 and the structure 7. Unwanted movement of the apparatus 10 in relation the structure 7 during measurements that might decrease the quality of the measurements can be avoided or a least greatly reduced.

In Figure 2, an exemplary propagation of ultrasonic waves 20 coming from the transmitter 15 is illustrated. The ultrasonic waves 20 first propagate through the water in which the apparatus 10 and the transmitter 15 are submerged until they hit the structure 7 at the surface area 7' which is formed by a first layer of the structure 7. In the present example, the first layer of the structure 7 is the steel wall of the monopile 2. Parts of the ultrasonic waves 20 are reflected while others enter the first layer (i.e. the steel wall of the monopile 2) and propagate further. Those waves propagating further then hit the interface between the first layer and the second layer, which - in this example - consists of grout 5. Again, parts of the ultrasonic waves 20 are reflected while others propagate further, and so on. All reflections either on outer surfaces of the structure 7 or coming from interfaces between the individual layers of the structure 7, again propagate through the water in which the apparatus 10 is submerged before they are detected by the detector 16 located on the apparatus 10. The detector 16 is thus on the same side of the investigated structure 7 as the transmitter 15.

The apparatus 10 is configured to transmit and detect ultrasonic energy in the full spectrum between 20 and 200 kHz. For this, the transmitter 15 is controlled by the analysing module 18 to continuously go through all frequencies between 20 and 200 kHz. The detector 16 detects the reflections for all said frequencies.

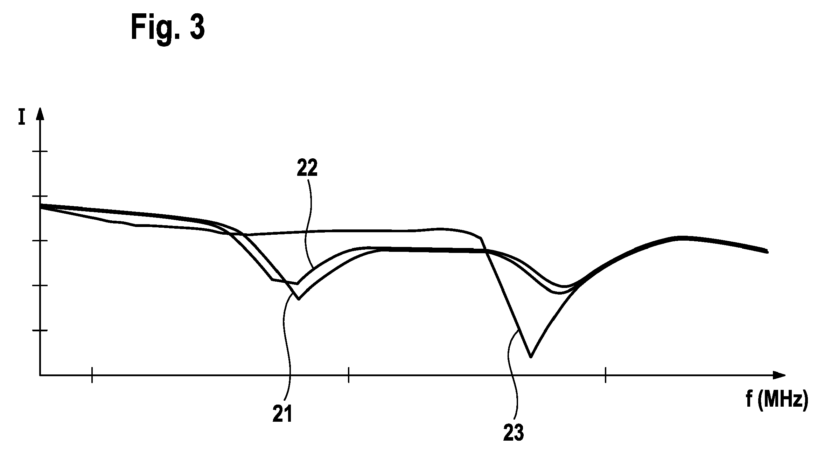

In Figure 3, an exemplary diagram of different test results received by using the apparatus 10 described above are shown. The data used for this diagram are provided by the analysis module 18. In the diagram the intensity of reflections over frequency is for illustrative purposes shown in a subrange of the above-mentioned frequency range only.

The curve designated with reference number 21 shows the intensity of reflections in case the structure 7 is fully undamaged. In comparison, curve 22 depicts the intensity of reflections in case of a de-bonding between the first and second layer of the structure, i.e. in the present case a de-bonding between the monopile 2 and the grout 5. Curve 23 shows the results in case of a crack with a width of 0,3 mm within the grout 5, wherein the crack is filled with seawater.

As can be seen from the exemplary diagram in Figure 3, different faults within the investigated structure 7 result in different and characteristic intensity distribution that can be categorized. In other words, each type of fault within a structure 7 will result in a specific and identifiable fingerprint in the reflection intensity over a frequency range. Depending on the resolutions, the fingerprints are also suitable for identifying the size of a fault, e.g. the width of a crack, or further information, e.g. whether the crack is filled with water or air.

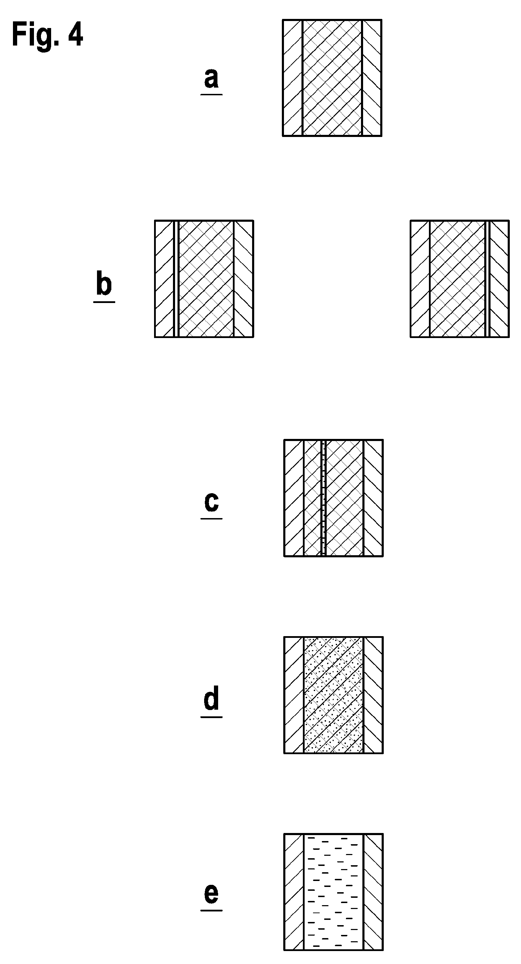

Figure 4 shows examples for various faults within a structure 7 that can be detected with the apparatus 10 shown in the previous figures. While Figure 4a shows the structure 7 in an undamaged state, figure 4b shows two possible debondings between the different layers of the structure 7. In Figure 4c, a crack within the grout layer of the structure 7 is depicted, where the crack is filled with water. The crack might, of course, also be filled with air. Figure 4d shows a fault of the structure 7, where the grout 5 has been granulized, i.e. the structural integrity of the grout 5 is greatly compromised. In Figure 4e, the grout 5 has been completely replaced by (sea)water.

All of the exemplary faults shown in figure 4 have their unique fingerprint in reflection intensity over frequency as described above. Same is true for any combination and varieties of the faults shown. Thus it is possible to identify the type of faults within a structure based on the detected reflections.

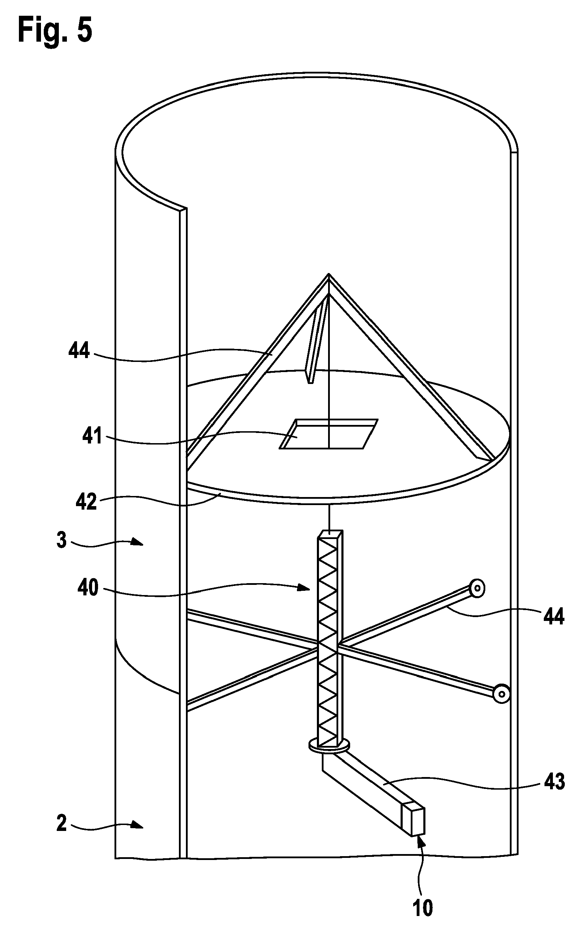

In Figure 5, a second embodiment of an apparatus 10 according to the invention is shown. In this embodiment, the apparatus 10 is not designed as a diving robot. Instead, an easily mountable and demountable frame 40 is lowered though a hatch 41 in an intermediate floor 42 at the top of the monopile 2. The apparatus 10 as such is located at a cantilever arm 43 at the bottom of the frame 40. The cantilever arm 43 can be rotated around the main axis of the frame 40. Hence, together with the possibility to adjust the height of the frame, each section of the overlap region between the monopile 2 and the transitional piece 3 with grout 5 (not shown in this figure) in between can be investigated by the apparatus 10. In order to provide sufficient stability the frame 40 comprises several guide arms 44 that butt against the monopile 2 or are affixed to the intermediate floor 42.

The frame 40 can be dismounted into parts that are sufficiently small to fit through openings and hatches typically present on offshore wind energy turbines.

**Claims**

1. Method for determining the condition of a building structure (7) with at least three layers, the structure comprising a layer of grout (5) between two layers of steel, in a wind turbine foundation (1), with continuous layers and seamless interfaces between the layers in undamaged condition, comprising the steps of

   - exposing a surface area (7') of the structure (7) to ultrasonic energy in a frequency range between 10 and 500 kHz;
   - detecting reflections from within the structure (7) at the side of the structure (7) on which the surface area (7') is; and
   - analysing the detected reflections to determine faults in the structure;

   wherein the ultrasonic energy comprises a spectrum of frequencies.

2. Method according to claim 1, wherein the layer of grout (5) has a nominal thickness between 50 and 90 mm, preferably between 60 and 80 mm, more preferably 70 mm and/or at least one of the layers of steel has a nominal thickness between 30 to 70 mm, preferably between 40 to 60 mm, more preferably of 50 mm.

3. Method according to any one of the preceding claims,
   wherein
   the faults in the structures (7) to be determined comprise the absence of a layer material, a crack at the interface between two layers and/or the decrease of structural integrity of one of the layers.

4. Method according to any one of the preceding claims,
   wherein
   the surface area of the structure (7) is submerged in water so that the ultrasonic energy is propagated through the water onto and from the surface area.

5. Method according to any one of the preceding claims,
   wherein the ultrasonic energy is propagated onto and/or from the surface area by compression waves.

6. Apparatus (10) for conducting the method according to any one of the previous claims, comprising

   - an ultrasonic transmitter (15) to expose ultrasonic energy onto a surface area (7') of a structure (7);
   - an ultrasonic detector (16) to detect reflections from within the structure (7) at the side of the structure on which the surface area (7') is; and
   - an data interface (17) for providing information about the detected reflections for analysis purposes;

   wherein the operational range of the transmitter (15) and the detector (16) comprises the frequencies between 10 and 500 kHz.

7. Apparatus according to claim 6, wherein the apparatus (10) comprises an analysis module (18) that is connected to the data interface (17) for at least partly analysing the detected reflections to determine faults in the structure (7).

8. Apparatus according to claim 6 or 7, wherein the apparatus (10) is submersible.

9. Apparatus according to any one of the claims 6 to 8, wherein the apparatus (10) comprises at least one spacer (19) for ensuring a predetermined distance between transmitter and/or detector and the surface area (7') of the structure (7) on which the ultrasonic energy is exposed.

10. Apparatus according to any one of the claims 6 to 9, wherein the apparatus (10) is a diving robot.

**Fig. 1**

# Fig. 2

EP 3 088 884 A1

## Fig. 3

# Fig. 4

# Fig. 5

Europäisches
Patentamt

European
Patent Office

Office européen
des brevets

# EUROPEAN SEARCH REPORT

Application Number

EP 16 16 7381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2002 195989 A (TANAKA SHOGO) 10 July 2002 (2002-07-10) * abstract; figures 1-3 * * paragraphs [0011] - [0029] * ----- | 1-10 | INV. G01N29/04 G01N29/11 G01N29/34 G01N33/38 |
| X | US 6 483 777 B1 (ZEROUG SMAINE [US]) 19 November 2002 (2002-11-19) * abstract; figures 1-4 * * paragraphs [0001] - [0020] * ----- | 6,7 | |
| X | JP 2005 148061 A (HIROSE MASAYUKI; NIPPON PS KK) 9 June 2005 (2005-06-09) * abstract; figures 1-4 * * column 6, line 44 - column 8, line 9 * ----- | 1,6,7 | |
| X | WO 2014/030615 A1 (IHI CORP [JP]; IHI INFRASTRUCTURE SYS CO LTD [JP]) 27 February 2014 (2014-02-27) * abstract; figure 1 * * paragraphs [0016] - [0018] * ----- | 1,6,7 | TECHNICAL FIELDS SEARCHED (IPC) G01N E02B |

The present search report has been drawn up for all claims

| Place of search | Date of completion of the search | Examiner |
|---|---|---|
| Munich | 2 September 2016 | Uttenthaler, Erich |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 3 088 884 A1**

## ANNEX TO THE EUROPEAN SEARCH REPORT
## ON EUROPEAN PATENT APPLICATION NO.

EP 16 16 7381

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2016

| Patent document cited in search report | | Publication date | Patent family member(s) | | Publication date |
|---|---|---|---|---|---|
| JP 2002195989 | A | 10-07-2002 | NONE | | |
| US 6483777 | B1 | 19-11-2002 | CA | 2316265 A1 | 15-07-1999 |
| | | | CN | 1292088 A | 18-04-2001 |
| | | | EP | 1047935 A1 | 02-11-2000 |
| | | | NO | 20003445 A | 04-09-2000 |
| | | | RU | 2213358 C2 | 27-09-2003 |
| | | | US | 6483777 B1 | 19-11-2002 |
| | | | WO | 9935490 A1 | 15-07-1999 |
| JP 2005148061 | A | 09-06-2005 | JP | 4640771 B2 | 02-03-2011 |
| | | | JP | 2005148061 A | 09-06-2005 |
| WO 2014030615 | A1 | 27-02-2014 | EP | 2889613 A1 | 01-07-2015 |
| | | | JP | 5909285 B2 | 26-04-2016 |
| | | | JP | WO2014030615 A1 | 28-07-2016 |
| | | | WO | 2014030615 A1 | 27-02-2014 |

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6483777 B